(19) **Europäisches Patentamt**

**European Patent Office**

**Office européen des brevets**

(11) Veröffentlichungsnummer: **0 208 061**

**A1**

(12) # EUROPÄISCHE PATENTANMELDUNG

(21) Anmeldenummer: **86104316.4**

(22) Anmeldetag: **27.03.86**

(51) Int. Cl.⁴: **A61M 1/34 , A61M 1/36**

(30) Priorität: **14.05.85 DE 3517362**

(43) Veröffentlichungstag der Anmeldung:
**14.01.87 Patentblatt 87/03**

(84) Benannte Vertragsstaaten:
**AT BE DE FR GB IT NL SE**

(71) Anmelder: **Biotest Pharma GmbH**
**Flughafenstrasse 4**
**D-6000 Frankfurt 71(DE)**

(72) Erfinder: **Handel, Klaus-Dieter, Dr.**
**Dr.techn.Dipl.-Ing.**
**Palleswiesenstrasse 14**
**D-6100 Darmstadt(DE)**
Erfinder: **Gänshirt, Karlheinz, Dr. Dipl.-Chem.**
**August-Bebel-Strasse 34**
**D-6072 Dreieich(DE)**

(74) Vertreter: **Wolff, Hans Joachim, Dr.jur.**
**Dipl.-Chem. et al**
**Beil, Wolff & Beil Rechtsanwälte Postfach 80**
**01 40 Adelonstrasse 58**
**D-6230 Frankfurt am Main 80(DE)**

(54) **Verfahren und Vorrichtung zur Gewinnung von Blutplasma.**

(57) Ein System zur Gewinnung von Blutplasma bei einer Blutentnahme vom lebenden Körper mit Blutentnahmekanüle (1) nach Stauung der entnahmefähigen Blutgefäße unter zusätzlicher Ausnutzung von äußerem Druck und Schwerkraft mit Hilfe einer Plasmafiltereinheit (4) als Trennvorrichtung bietet ein Optimum an Sicherheit für den Spender und an Funktionstüchtigkeit, wenn man Vorfüllungs-Lösung - (10) mit Hilfe eines separaten Anschlusses (2) zugibt, das gespendete Blutvolumen mißt, die Stabilisatorlösung (7) mit Hilfe einer Pumpe (8) entsprechend dem gemessenen gespendeten Blutvolumen zudosiert, das Blut durch eine zwischen Blutentnahmestelle (1) und Plasmafiltereinheit (4), jedoch nach dem Anschluß (11) für die Stabilisatorlösung (7) liegende Tropfkammer (6) mit eingebautem Transfusionsfilter führt und die Sammelstelle (5) für Plasma während des ganzen Verfahrens tiefer als Sammelstelle (3) für Blut anordnet, oder Blut in einen mit Stabilisatorlösung gefüllten Beutel (13) spendet, den vom Beutelsystem (12) abgetrennten Beutel (13) an oberes Ende eines Transfusionssystems (20) anschließt und Blut über Plasmafiltereinheit (4) und Tropfkammer (6) mit Transfusionsfilter retransfundiert und während der Retransfusion Plasma sammelt.

Fig. 2

## Verfahren und Vorrichtung zur Gewinnung von Blutplasma

Die Erfindung betrifft ein Verfahren zur Gewinnung von Blutplasma nach dem Oberbegriff des Anspruchs 1 bzw. 4 sowie eine Vorrichtung zur Durchführung des Verfahrens.

Bei der routinemäßig mit Beutel durchgeführten Plasmaspende spendet der Spender eine bestimmte Menge Blut in einen Beutel, in dem eine auf die Blutmenge abgestimmte Menge Stabilisatorlösung bzw. Antikoagulanzlösung für Blut vorliegt. Nach der Spende wird der Beutel abgetrennt und die Bildung von Gerinnsel in der Venenkanüle im Arm des Spenders durch langsames Infundieren von isotoner NaCl-Lösung über ein Infusionsbesteck verhindert. Der Beutel wird dann zentrifugiert, um das Plasma von den Blutzellen abzutrennen, was meist in einem separaten Raum erfolgt. Anschließend werden die Blutzellen mit etwas isotoner NaCl-Lösung verdünnt und dem Spender über das Infusionsgerät retransfundiert. Dies wird in der Regel zweimal hintereinander durchgeführt, und man erhält so etwa 500 -600 ml Plasma. - ("Richtlinien fur Plasmapheresen", Deutsches Ärzteblatt, Heft 5 (1977) 305-314; "Handbuch der Blutkomponenten-Therapie" American Association of Blood Banks (1969) 24-33)

Die Zentrifugen fur das Zentrifugieren des Plasmas sind sehr groß und schwer und brauchen zur Energieversorgung einen Netzstromanschluß. Somit kann eine Plasmaspende nur in einem eingerichteten Spendezentrum durchgefuhrt werden.

Da zum Zwecke des Zentrifugierens das gespendete Blut vom Spender entfernt werden muß, kann es trotz aller Vorsichtsmaßnahmen zu Verwechslungen kommen, und dem Spender können falsche Blutzellen retransfundiert werden ("Manuel of Blood Component Preparation", American Association of Blood Banks (1969) 39-44).

Es war nun wünschenswert, auch bei Blutspendeterminen, die nicht in den Spendezentren stattfinden, Plasmaspenden durchführen zu können. Der Bedarf an Plasma ist größer als durch Blutspenden abgedeckt werden kann. Außerdem belastet eine Plasmaspende das blutbildende System weniger als eine Blutspende, so daß eine wöchentliche Spende von 500 ml Plasma möglich ist, während eine Blutspende von 500 ml Blut nur alle 12 Wochen erfolgen kann. Man versuchte ferner ein Verfahren zu finden, das das Wegtransportieren des Spenderblutes nicht mehr erforderlich macht.

Es wurden deshalb Verfahren und Vorrichtungen zur Gewinnung von Blutplasma bei einer Blutentnahme am lebenden Körper unter Verwendung einer Plasmafiltereinheit entwickelt, bei denen das Spenderblut mit Hilfe von Schlauchpumpen zur Plasmafiltereinheit und durch diese hindurchgepumpt werden. Derartige Systeme sind jedoch sehr aufwendig und erfordern an mehreren Stellen Überwachungseinrichtungen für Drücke, Strömungen und Luftblasen.

Aus der DE-OS 33 02 383 bzw. Lysaght, M. et al.: "Plasmapheresis without pumps..."; Int.Workshop Plasma Separation and Plasma Fractionation 17.-19. März 1983, Rottach-Egern, Seiten 1-14, sind ein Verfahren zur Gewinnung von Blutplasma bei einer Blutentnahme nach dem Oberbegriff des Anspruchs 1 bzw. 4 und eine Vorrichtung nach dem Oberbegriff des Anspruchs 7 bzw. 8 bekannt.

Dieses Verfahren arbeitet ohne Blutpumpen, Tropfkammern oder andere Apparaturen, sondern unter Ausnutzung der Schwerkraft und ist somit wesentlich einfacher und weniger aufwendig als bisherige mit Pumpen und anderen Apparaturen arbeitende Verfahren. Es wird dort lediglich unter Verwendung einer Blutentnahmekanüle nach einer Stauung der entnahmefähigen Blutgefäße Spenderblut mittels eines Schlauches zu einer tiefer gelegenen Plasmafiltereinheit geleitet, worin Plasma und Blut getrennt und einer Sammelstelle für Plasma und einer davon getrennten Sammelstelle für Blut zugeleitet werden. Zwischen Blutentnahmestelle und Plasmafiltereinheit befindet sich ein Anschluß für eine Antikoagulanzlösung.

Bei diesem System braucht der Blutbeutel nicht zum Zwecke des Zentrifugierens wegtransportiert zu werden, sondern bleibt bei der Spende, wodurch eine geringere Verwechselungsgefahr besteht.

Neben dem Vorteil der einfachen Handhabung und der geringeren Verwechselungsgefahr weisen dieses Verfahren und die Vorrichtung jedoch einige Nachteile auf.

Das System sieht keine Messung des Spendevolumens vor, wobei die Gefahr besteht, daß ein zu großes Blutvolumen sich außerhalb des Körpers des Spenders befindet, wobei es zu einem Kreislaufkollaps kommen kann. Die Antikoagulanzlösung wird dem System in einer konstanten Menge zugeführt, jedoch unabhängig vom Blutfluß, nachdem dieser nicht gemessen wird. Der zu erwartende Blutfluß ist vor der Spende nicht bekannt und kann sich außerdem während der Spende ändern. Aus diesem Grunde kann Überdosierung oder Unterdosierung der Antikoagulanzlösung erfolgen. Überdosierung bedeutet bei der Retransfusion fur den Spender eine zusätzliche, möglicherweise mit Komplikationen verbundene Belastung. Sehr unangenehm kann es für den Spender werden, wenn der Blutfluß stoppt wegen einer Verstopfung nach der Antikoagulanz-Zufuhrstelle. In diesem Falle läuft die Antikoagulanz-Zufuhr weiter, und zwar in die Vene des Spenders. Es wird also reine Antikoagulanzlösung infundiert, wovor auf dem Aufdruck von Behältern mit Antikoagulanzlösung ausdrucklich gewarnt wird. Unterdosierung fördert die Bildung von Mikroaggregat und birgt die Gefahr der Gerinnungsaktivierung in sich, was bei Einsatz von Membranfiltern zur Plasmaabtrennung insofern besonders kritisch ist, als hier eine große Fremdoberfläche für das Blut vorliegt und die Gefahr einer Gerinnungsaktivierung besonders gegeben ist.

Vor Beginn der Spende muß das System mit isotoner Lösung vorgefüllt werden. Ist das System nicht von Anfang an vorgefüllt, so kann der Transmembrandruck (TMP) sogar negativ sein, wenn bei langsamem Blutfluß das für die Strömung notwendige Druckgefälle zwischen Plasmafiltereinheit und Blutsammelbeutel kleiner wird als die hydrostatische Druckdifferenz. Es findet dann keine Plasmafiltration statt. Es muß daher die Leitung zum Plasmabeutel mit Flussigkeit gefüllt sein, um einen zusätzlichen Unterdruck aufgrund der Höhendifferenz zwischen Plasmafiltereinheit und Plasmabeutel sicherzustellen, so daß auf jeden Fall ein positiver TMP vorliegt, d.h. daß der Druck auf der Blutseite der Membran großer ist als auf der Plasmaseite.

Die Luft muß aus den Schlauchleitungen weitgehend entfernt werden, um bei der Retransfusion die Gefahr einer Luftembolie zu vermeiden. Beim bekannten System wird deshalb vor der Spende mit isotoner NaCl-Lösung über die Entnahmekanüle gespült. Beim Vorfüllen des Systems über die Blutentnahmekanüle kann letztere jedoch beim Einstechen in den Stopfen einer Flasche mit NaCl-Lösung verändert werden und eine Venenpunktion dann erschwert werden.

Wie bereits vorstehend ausgeführt, arbeitet das System ohne Tropfkammer und ohne Infusionsfilter, was, wie nachstehend genauer erläutert wird, sehr gefährlich sein kann.

Schließlich befinden sich die Sammelstelle für Plasma und die Sammelstelle für Blut bei der Blutspende praktisch auf einer Ebene. Mit einer solchen Anordnung kann über die kommunizierenden Schlauchleitungen bei Stillstand des Blutflusses das Plasma über die Plasmafiltereinheit in die Sammelstelle für Blut fließen, falls der Plasmabeutel geringfügig über dem Blutbeutel hängt, was sehr leicht auftreten kann.

Der Erfindung liegt die Aufgabe zugrunde, ein Verfahren und eine Vorrichtung zur Gewinnung von Blutplasma bei einer Entnahme vom lebenden Körper bereitzustellen, die leicht zu handhaben und einfach sind und sich keiner aufwendigen Blutpumpen und sonstiger Apparaturen bedienen, sondern unter Ausnutzung der Schwerkraft arbeiten, dabei jedoch ein Optimum an Sicherheit für den Spender bieten und keine Funktionsmängel aufweisen.

Diese Aufgabe wird bei einem gattungsgemäßen Verfahren durch die kennzeichnenden Merkmale des Anspruchs 1 bzw. 4 und bei einer gattungsgemäßen Vorrichtung durch die kennzeichnenden Merkmale des Anspruchs 7 bzw. 8 gelöst.

Nach einer Ausführungsform der Erfindung wird die Stabilisator-bzw. Antikoagulanzlösung mit Hilfe einer Dosierpumpe entsprechend dem gemessenen Blutvolumen genau dosiert, wodurch ein optimales Stabilisator/Blut-Verhältnis eingehalten wird und weder durch Unterdosierung die Gerinnungsaktivierung begünstigt wird, noch durch Überdosierung der Spender bei der Spende gefährdet oder bei der Retransfusion zusätzlich belastet wird.

Um zu vermeiden, daß ein zu großes Volumen an Blut sich außerhalb des Körpers befindet und es somit zu einem Kreislaufkollaps kommen kann, wird erfindungsgemäß das gespendete Blutvolumen laufend gemessen. Die Messung erfolgt vorzugsweise mit einem Ultraschalldetektor an der Außenseite des blutführenden Schlauches. Eine geeignete Meßmethode wird in der EP-OS 83 100 079 beschrieben. Eine weitere, jedoch nicht so bevorzugte Meßmethode besteht in der Verwendung einer elektronischen Waage.

Als Dosierpumpe für Antikoagulanzlösung läßt sich jede Art von auf dem Markt befindlichen Dosierpumpen verwenden. Besonders brauchbar sid Peristaltikpumpen, wie sie für diesen Zweck in der EP-OS 83 100 079 beschrieben werden.

Als ganz besonders brauchbar erwies sich ein Blutentnahmegerät (BEG 404) der Fa.Doltron AG. Bei diesem Gerät wird der Blutfluß mit einem Ultraschalldetektor gemessen und die Antikoagulanzlösung über eine Perestaltikpumpe dem Blutfluß entsprechend zudosiert. Aus dem Blutfluß wird das Blutvolumen berechnet und angezeigt, wenn ein vorbestimmtes Volumen erreicht ist.

Es ist ferner nicht unbedingt erforderlich, die Vorfüllungs-Lösung am Anfang des Systems mit Hilfe der Blutentnahmekanüle einzufüllen, sondern dies ist auch mit Hilfe eines separaten Anschlusses an irgendeiner Stelle des Systems möglich. Erfindungsgemä8 ist deshalb ein separater Anschluß für die Vorfullungs-Lösung vorgesehen. Dadurch wird die Entnahmekanüle geschont, und die Venenpunktion wird nicht durch eine bereits vorher schon einmal eingestochene Kanüle erschwert.

Der Anschluß für die Vorfüllungs-Lösung kann mit einer Kanüle oder einem Dorn versehen sein zum Einstechen in eine mit Vorfüllungs-Lösung gefullte Flasche oder einen Beutel.

Während der Retransfusion können sich aus dem System Luftblasen lösen, die beim Einfüllen mit isotoner Lösung und während der Blutspende nicht vollständig entfernt wurden (Gefahr der Luftembolie). Zum anderen besteht immer die Möglichkeit, daß im System trotz ausreichender Zugabe von Antikoagulanzlösung Gerinnungsvorgänge ablaufen, die zur Bildung von Mikroaggregaten führen können. Diese müssen bei der Retransfusion der Erythrozyten herausgefiltert werden (Gefahr der Thrombose). Aus Sicherheitsgründen, um diese Gefahren auszuschließen, ist erfindungsgemäß eine Luftblasenfalle mit eingebautem Transfusionsfilter vorgesehen. Diese Falle befindet sich hinter dem Anschluß für die Antikoagulanzlösung und vor der Plasmafiltereinheit, bezogen auf den Blutfluß während der Blutspende. Eine solche Luftblasenfalle mit Transfusionsfilter liegt in Form einer Tropfkammer mit Filter für Blut und Blutbestandteile vor, beispielsweise wie sie gemäß DIN 58 360 Teil 1 beschrieben wird.

Schließlich wurde gefunden, daß es zur Vermeidung eines Überfließens von Plasma aus der Sammelstelle für Plasma zur Sammelstelle für Blut, bei Stillstand des Blutflusses, der z.B. bei Verlegen der Venenkanüle durch die Venenwand eintritt, erforderlich ist, sowohl während der Blutspende als auch während der Retransfusion die Sammelstelle für Plasma tiefer als den Blutbeutel zu hängen.

Nach einer weiteren, bevorzugten Ausführungsform der Erfindung erfolgt die Abtrennung des Plasmas nicht mehr während der Blutspende, sondern nur noch während der Retransfusion.

Dabei erfolgt die Spende in einen Blutbeutel, dem eine bestimmte, auf die zu spendende Blutmenge abgestimmte Stabilisatorlösung vorliegt, auf ähnliche Weise, wie in der vorstehend genannten Literatur zur Plasmapherese beschrieben wird. Danach wird der Beutel vom Beutelsystem abgetrennt und an das obere Ende eines Transfusionsgerätes angeschlossen, das mehrere Einstichdorne am oberen Ende, eine Plasmafiltereinheit, eine Sammelstelle für Plasma und eine Tropfkammer mit eingebautem Transfusionsfilter enthält und mit dem unteren Ende mit dem Beutelsystem und somit der Spenderkanüle verbunden ist.

Das gespendete Blut kann nun über die Plasmafiltereinheit dem Spender retransfundiert werden. Während das Blut durch die Plasmafiltereinheit fließt, wird das Plasma abgetrennt und in einem Plasmasammelbeutel aufgefangen.

Diese bevorzugte Ausführungsform ist noch sicherer und noch einfacher als die vorstehend beschriebene Ausführungsform der Erfindung.

Sicherer deshalb, weil beim Durchströmen des Transfusionsfilters in beiden Richtungen Stoffe, z.B. Mikroaggregate, die bei der Blutentnahme im Filter zurückgehalten wurden, beim umgekehrten Durchströmen doch mal in den Spender retransfundiert werden können und weil diese Gefahr beim einfachen Durchströmen, d.h. nur beim Retransfundieren, ausgeschlossen ist.

Einfacher deshalb, weil kein Dosiersystem für die Stabilisatorlösung erforderlich ist und jeglicher apparativer Aufwand, wie die Dosierpumpe als solche und Leitungs-oder Batteriestrom, entfallen. Das Stabilisator-bzw. Antikoagulanzproblem ist mit dieser Ausführungsform total gelöst.

Zur Gewinnung einer gewissen Plasmamenge sind zwar mehr Zyklen erforderlich als bei der ersten Ausführungsform, weil nur während der Retransfusion Plasma gewonnen wird, d.h. die Plasmaextraktion pro Volumeneinheit Blut ist etwas geringer, dafür ist aber die Zykluszeit kürzer, weil der Hämatokritwert niedriger ist.

Die Retransfusionszeit kann außerdem noch dadurch verkürzt werden, daß man um den gefüllten Beutel eine Druckmanschette legt, wie sie bei Drucktransfusion üblich ist.

Das Verfahren kann mehrere Male wiederholt werden, bis eine ausreichende Plasmamenge gespendet worden ist.

Am Beutelsystem befindet sich mindestens ein Blutbeutel, vorzugsweise drei, jedoch sind bis zu fünf Beutel möglich. Dementsprechend sollte auch die Anzahl der Einstichdorne am oberen Ende des Transfusionsgerätes sein. D.h. es sollten mindestens so viele Einstichdorne vorhanden sein, wie anzuschließende Blutbeutel vorgesehen sein können plus ein Einstichdorn für den Anschluß der Vorfüllungs-Lösung.

Als Vorfüllungs-Lösung wird vorzugsweise eine isotone NaCl-Lösung verwendet.

Stabilisatorlösungen werden in der Literatur beschrieben. Zu den üblichen Lösungen gehören beispielsweise Na-Citrat-und CPD-(Citrat-Phosphat-Dextrose)Lösungen.

Als Plasmafiltereinheit lassen sich Module sowohl mit Flachmembranen als auch mit Hohlfasermembranen verwenden. Die Porengröße der Membranen sollte zweckmäßigerweise 0,2 bis 0,8 $\mu$m, vorzugsweise maximal 0,65 $\mu$m betragen. Bei einer zu kleinen Porengröße, z.B. unter 0,2 $\mu$m, könnten Proteine zurückgehalten werden. Der Innendurchmesser von Hohlfasermembranen kann beliebig sein, jedoch werden durch das extrakorporale Blutvolumen Grenzen gesetzt. Deshalb sollte der Innendurchmesser nicht kleiner als 100 μm und nicht größer als 500 μm sein.

Eine brauchbare Plasmafiltereinheit mit Hohlfasermembran ist die Plasmapur(®)-Patrone, wie sie im Firmenprospekt "Pure plasma..... Plasmapur(®)" der Firma Organon Technika beschrieben wird. Ebenfalls brauchbar ist das Travenol CPS-10 Plasmafilter mit einer aktiven Hohlfaserlänge von 21 cm, wie es in der DE-OS 33 02 383 beschrieben wird.

Eine geeignete Plasmafiltereinheit mit Flachmembran wird beispielsweise in der DE-PS 29 25 143 beschrieben.

Als Sammelstelle für Blut und für Plasma lassen sich Blutflaschen oder Blutbeutel aus Kunststoff verwenden, wie sie allgemein zur Aufbewahrung von Blut üblich sind. Die Verbindungsschläuche zwischen den einzelnen Elementen der erfindungsgemäßen Vorrichtung können ebenfalls übliche Kunststoff-oder Gummischläuche sein, wie sie bei Transfusionsgeräten üblich sind und z.B. den Forderungen gemäß DIN 58 360 oder DIN 58 367 entsprechen. Derartige Schläuche haben einen Innendurchmesser von mindestens 3 mm.

Nachstehende Zeichnungen dienen der weiteren Erläuterung der Erfindung.

Es zeigen:

Fig. 1 eine erfindungsgemäße Vorrichtung in erfindungsgemäßer Anordnung zur Blutspende;

Fig. 2 eine erfindungsgemäße Vorrichtung in erfindungsgemäßer Anordnung zur Retransfusion;

Fig. 3 eine Vorrichtung ohne Anschluß an eine Vorfüllungs-Lösung in Anordnung zur Blutspende, wobei die Sammelstelle für Blut tiefer hängt als die Sammelstelle für Plasma;

Fig. 4 eine Vorrichtung ohne Anschluß an eine Vorfüllungs-Lösung in Anordnung zur Retransfusion, wobei die Sammelstelle für Blut höher hängt als die Sammelstelle für Plasma;

Fig. 5 eine bevorzugte Vorrichtung gemäß der Erfindung;

Fig. 6 eine stark vereinfachte Vorrichtung zur Effektivitätsmessung des bevorzugten Systems.

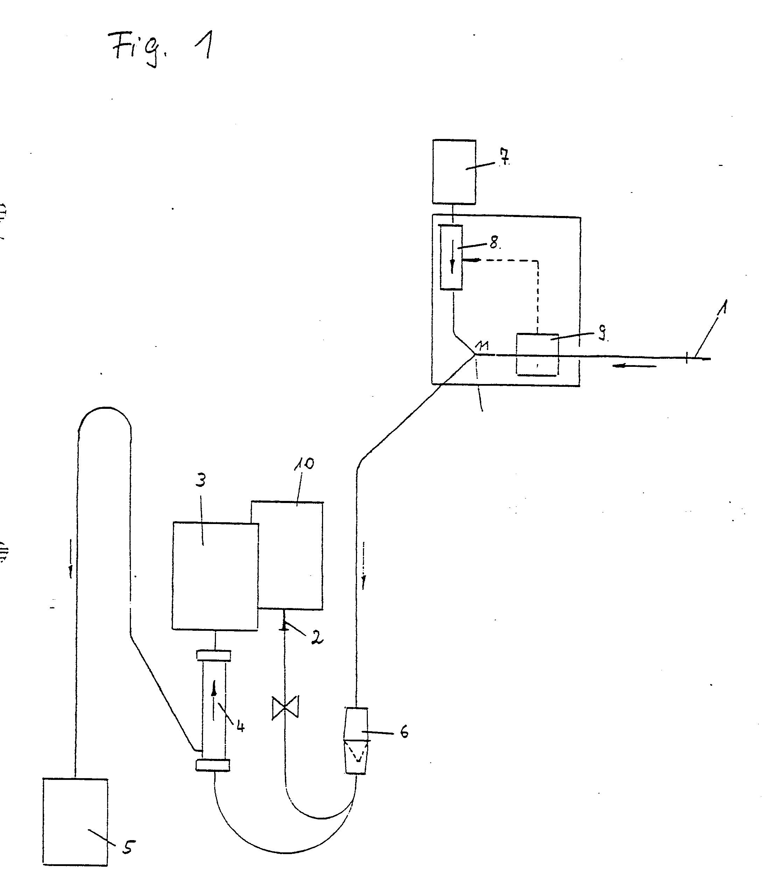

In Fig. 1 wird Blutentnahmekanüle (1) gezeigt, von der aus Blut durch ein Schlauchsystem, das mit einer Meßvorrichtung (9) für gespendetes Blutvolumen versehen ist, durch Tropfkammer mit eingebautem Transfusionsfilter (6) zur Plasmafiltereinheit (4) fließt. Zwischen Tropfkammer (6) und Blutentnahmekanüle (1) befindet sich der Anschluß - (11) an Stabilisatorlösung (7), die mit Hilfe der Dosierpumpe (8) dem System in Abhängigkeit vom gespendeten Blutvolumen zudosiert wird. Beim Durchströmen durch die Plasmafiltereinheit (4) wird Plasma vom Blut getrennt und fließt zur Sammelstelle für Plasma (5), während Blut zur Sammelstelle für Blut (3) fließt. Der Anschluß (2) für die Vorfüllungs-Lösung (10) befindet sich bei dieser Ausführungsform zwischen Tropfkammer (6) und Plasmafiltereinheit (4), kann sich jedoch an jeder anderen beliebigen Stelle des Systems befinden, vorzugsweise zwischen Blutentnahmekanüle (1) und Plasmafiltereinheit (4). Ebenso kann sich die Meßvorrichtung (9) an irgendeiner beliebigen Stelle zwischen (1) und (4) befinden. Bei der Blutspende sind Blutentnahmekanüle (1) und Anschluß (11) mit Stabilisatorlösung (7) und Dosierpumpe (8) oberhalb der übrigen Elemente des Systems angeordnet, damit Blut mit Hilfe der Schwerkraft fließen kann. Die Sammelstelle für Plasma (5) befindet sich unterhalb der Sammelstelle für Blut (3).

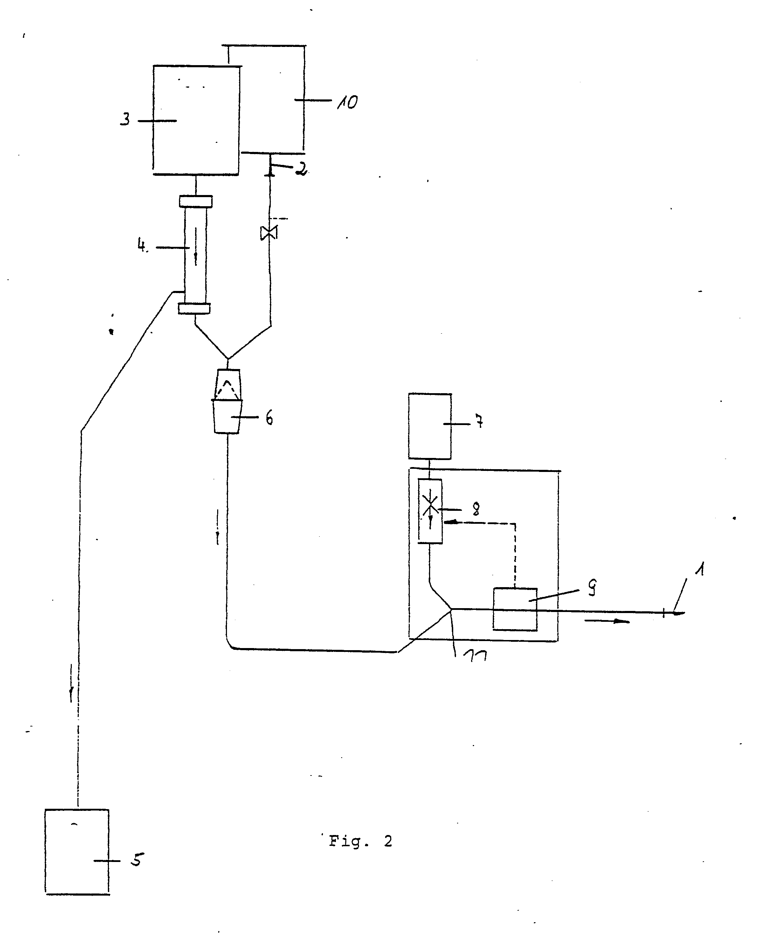

In Fig. 2 haben alle Elemente die gleiche Bedeutung wie in Fig. 1. Die Fig. 2 unterscheidet sich von Fig. 1 lediglich dadurch, daß zum Zwecke der Retransfusion diesmal Sammelstelle für Blut (3), Plasmafiltereinheit (4) und Tropfkammer (6) höher als die Blutentnahmekanüle angeordnet sind, damit das nur zum Teil vom Plasma befreite Blut mit Hilfe der Schwerkraft zum Spender zurückfließen kann. Bei diesem Zurückfließen passiert das partiell deplasmatisierte Blut zum zweiten Mal die Plasmafiltereinheit (4) und eine weitere Plasmamenge wird vom Blut abgetrennt. Während der Retransfusion fördert die Dosierpumpe (8) keine Stabilisatorlösung (7).

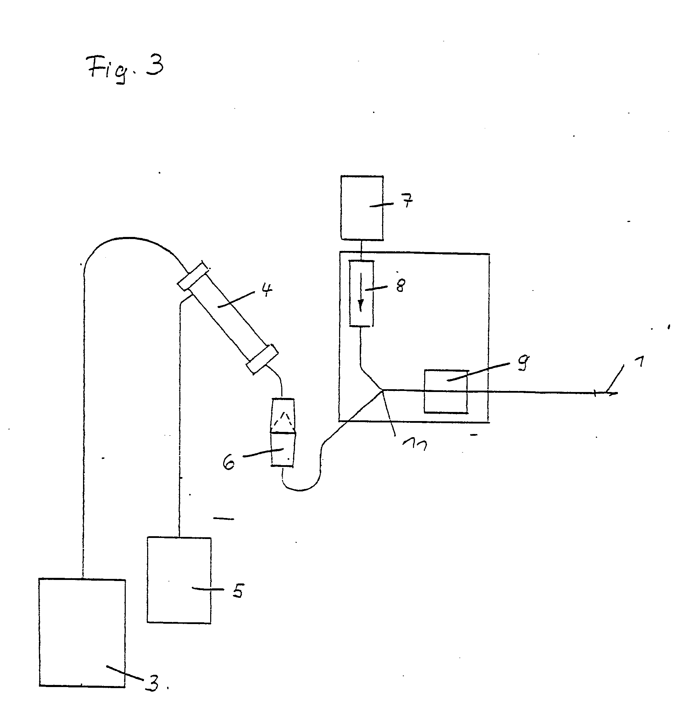

In Fig. 3 haben ebenfalls alle Elemente die gleiche Bedeutung wie in Fig. 1. Fig. 3 unterscheidet sich von Fig. 1 dadurch, daß kein Anschluß (2) für Vorfullungs-Lösung (10) vorgesehen ist und die Sammelstelle fur Blut (3) tiefer angeordnet ist als die Sammelstelle für Plasma (5).

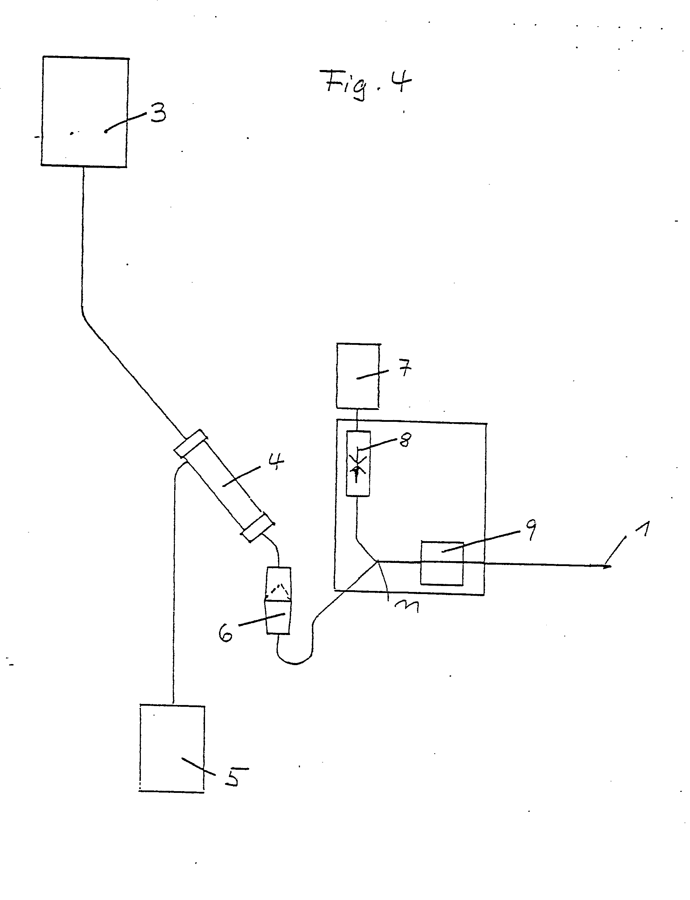

In Fig. 4 haben ebenfalls alle Elemente die gleiche Bedeutung wie in Fig. 1. Fig. 4 unterscheidet sich von Fig. 2 dadurch, daß kein Anschluß (2) fur Vorfullungs-Lösung (10) vorgesehen ist.

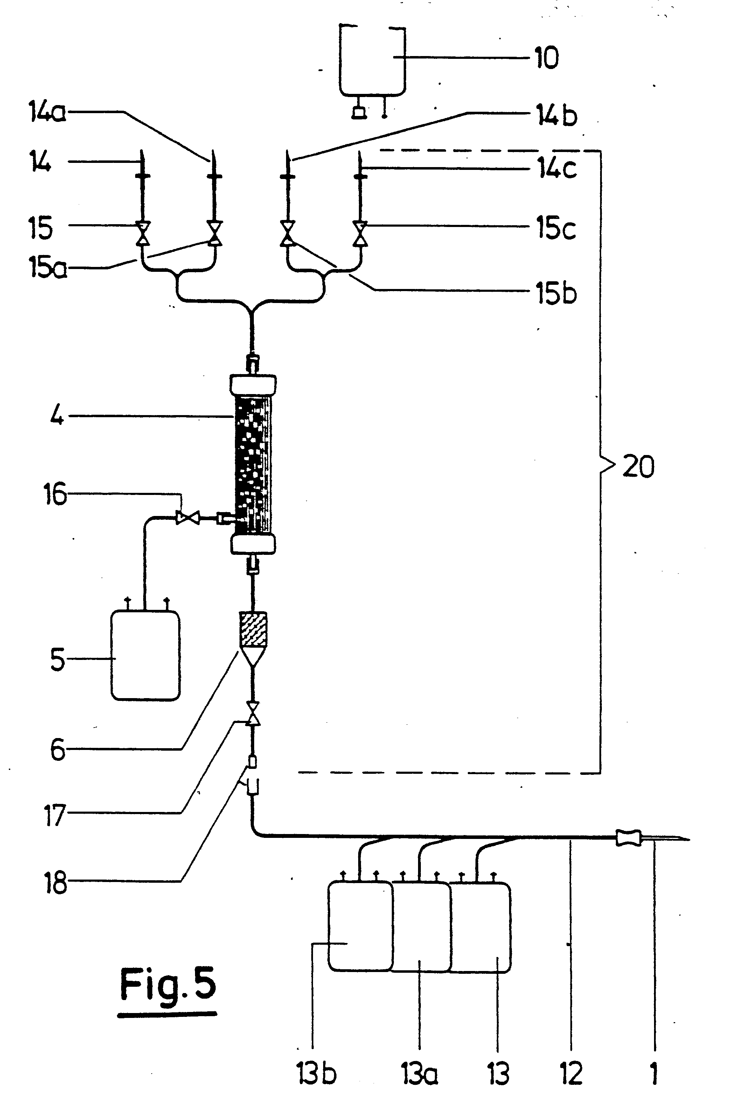

In Fig. 5 wird eine bevorzugte Vorrichtung gezeigt, bei der die Plasmagewinnung nur während der Retransfusion erfolgt.

Es wird dort Blutentnahmekanüle (1) gezeigt, von der das Spenderblut in das Beutelsystem (12) und in die Beutel (13, 13a, 13b) fließt. Es wird ferner das Transfusionsgerät (20) gezeigt, das die Plasmafiltereinheit (4), Sammelstelle für Plasma (5), Tropfkammer mit eingebautem Transfusionsfilter - (6), Einstichdorne (14,14a,14b) zum Anschluß an die Blutbeutel (13,13a,13b), Einstichdorn (14c) zum Anschluß an die Vorfüllungs-Lösung (10) und Rollklemmen (15,15a,15b,16,17) enthält. Blutbeutelsystem (2) und Transfusionsgerät (20) weisen jeweils an einem Ende ein Verbindungssystem (18) auf, mit Hilfe dessen sie miteinander verbunden werden können.

Nachdem diese Verbindung erfolgt ist, wird das gesamte System über Einstichdorn (14c) mit der Vorfullungs-Lösung (10) vorgefüllt. Danach beginnt die Punktierung des Spenders mit Hilfe der Blutentnahmekanüle (1). Nachdem die mit Stabilisatorlösung vorgelegten Blutbeutel (13) mit Spenderblut gefüllt sind, werden sie vom Beutelsystem - (12) abgeklemmt und abgetrennt und mit Hilfe der Einstichdorne (14,14a,14b) an das höher liegende Transfusionssystem angeschlossen. Nun erfolgt die Retransfusion, bei der das Blut mit Hilfe von äußerem Druck und Schwerkraft durch Plasmafiltereinheit (4) fließt, wobei das Plasma abgetrennt und in der Sammelstelle fur Blutplasma (5) gesammelt wird. Das deplasmatisierte Blut fließt dann weiter durch die Tropfkammer mit eingebautem Transfusionsfilter (6) zurück zum Spender.

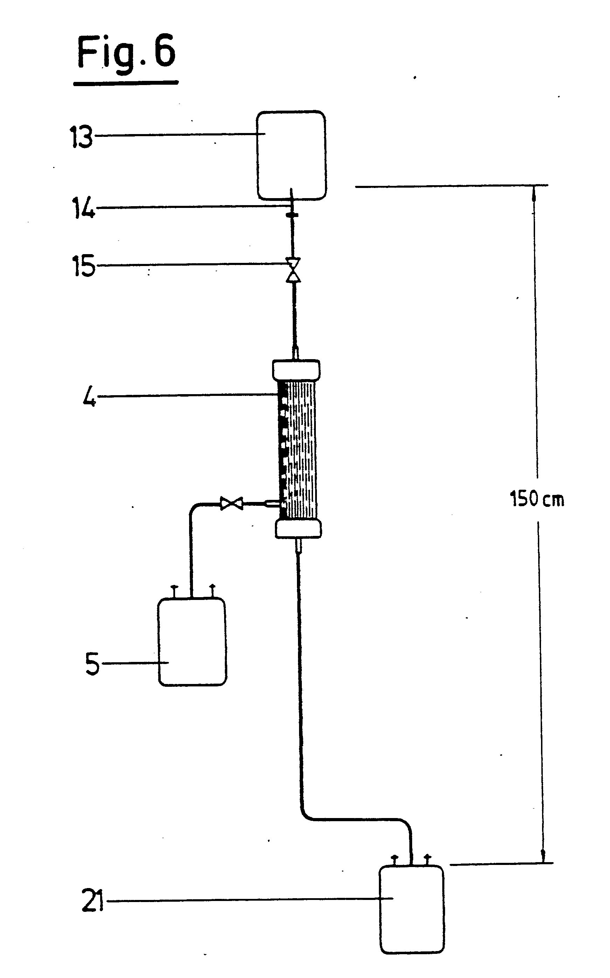

In Fig. 6 wird eine stark vereinfachte Vorrichtung gezeigt, die dazu dienen soll, die Effektivität des bevorzugten Systems beim Durchströmen des stabilisierten Blutes durch eine Plasmafiltereinheit zu messen, ohne direkte Verbindung mit dem Spender.

Man sieht dort einen mit stabilisiertem Blut gefüllten Blutbeutel (13), Einstichdorn (14), Rollklemme (15), Plasmafiltereinheit (4), Sammelstelle für Plasma (5) und einen ca. 150 cm tiefer als der Blutbeutel (13) angeordneten Leerbeutel (21) zum Sammeln des durch die Plasmafiltereinheit (4) geflossenen Blutes.

Nachstende Beispiele dienen der weiteren Erläuterung der Erfindung.

Beispiel 1

Ein System bestehend aus:

Kanüle (Plasmapheresekanüle Fa. Biotest)

Tropfkammer mit Transfusionsfilter (TNSB-Gerät Fa.Biotest)

Hollow fiber Modul zur Plasmaabtrennung - (Plasmapur der Fa. Organon Technika)

Blutbeutel zum Sammeln des Blutes (Leerbeutel 500 ml Fa. Biotest)

Plasmabeutel zum Sammeln des Plasma - (Leerbeutel 500 ml Fa. Biotest)

Beutel mit Blutstabilisator (Na-Zitrat pH = 7. Fa. Biotest) wurde gemäß Fig. 3 mit PVC-Schläuchen verbunden.

Die Dosierung der Stabilisatorlösung erfolgte mit einem Blutentnahmegerät (BEG 404) der Fa. Doltron.

Der Blutbeutel hing tiefer als der Plasmabeutel. Ein Blutspender spendete 500 ml Blut direkt durch die Trenneinheit in den Blutsammelbeutel, ohne daß das System mit isotoner NaCl-Lösung vorgefüllt war. Das System füllte sich zu Beginn nur sehr langsam mit Blut, da die Schläuche nicht mit Flüssigkeit gefüllt waren, so daß das hydrostatische Druckgefälle noch nicht den Blutfluß unterstützen konnte.

Sobald das Blut die Trenneinheit gefullt hatte, begann Plasma durch die Membrane zu treten und sammelte sich im Plasmabeutel. Der Blutfluß wurde zeitweilig durch Verlegen der Kanüle unterbrochen. Das Plasma strömte aus dem Plasmabeutel über die Trenneinheit in den Blutbeutel. Die Plasmaleitung war dann mit der Luft des Plasmabeutels gefüllt. Nach Korrektur der Lage der Kanüle in der Vene konnte der Blutfluß zwar wieder hergestellt werden, es trat jedoch keine Plasmafiltration mehr auf, da in der Plasmaleitung keine Flüssigkeitssäule mehr vorhanden war.

Beispiel 2

Es wurde das Beispiel 1 wiederholt, jedoch wurde das System mit isotoner NaCl-Lösung vorgefüllt und der Plasmasammelbeutel tiefer als der Blutsammelbeutel angebracht. Das Füllen des Systems mit isotoner NaCl-Lösung gestaltete sich - schwierig, da keine entsprechenden Anschlüsse hierfur vorgesehen waren. Es mußten Verbindungen geöffnet werden, um die Vorfüllungs-Lösung anschließen zu können und nach dem Fullen wieder umgesteckt werden.

In 8 Minuten wurden 500 ml Blut gespendet und dabei 160 ml Plasma gesammelt. Der Rückfluß des Blutes bei geänderter Schwerkraftlage (Fig. 4) in einen anstelle der Venenkanüle angeschlossenen Leerbeutel dauerte 7 Minuten, und es wurden weitere 80 ml Plasma gesammelt. In einem Zyklus konnten somit aus 500 ml Blut 240 ml Plasma gewonnen werden.

Beispiele 3 und 4

In das Schlauchsystem der Fig. 3 bzw. 4 wurde ein Anschluß für die Vorfüllungs-Lösung gemäß Fig. 1 bzw. 2 eingebaut. Die Komponenten entsprachen den Beispielen 1 bzw. 2.

Das System wurde mit isotoner NaCl gefüllt. Bei dieser Anordnung konnte durch Drücken auf die Tropfkammer die Tropfkammer selbst, die Plasmafiltereinheit und der Schlauch zwischen der Plasmafiltereinheit und dem Plasmasammelbeutel gefüllt werden, ohne das System an einer Stelle öffnen zu mussen. Die restliche Luft, die sich im Schlauch zwischen der Blutentnahmekanüle und der Tropfkammer befand, sammelte sich bei der Blutspende in der Tropfkammer. Die Beispiele wurden analog des Beispiels 2 durchgeführt.

|  | Beispiel 3 | Beispiel 4 |
|---|---|---|
| Spendezeit für 500 ml Blut | 9 Min. | 11 Min. |
| gesammeltes Plasma | 140 ml | 125 ml |
| Zeit für den Rückfluß des Blutes in einen Leerbeutel | 8 Min. | 8 Min. |
| zusätzlich gesammeltes Plasma | 80 ml | 75 ml |
| gesamte Plasmamenge | 220 ml | 200 ml |

Aus den Beispielen 1 und 2 ist ersichtlich, daß es zwingend notwendig ist, das System mit einer Vorfüllungs-Lösung zu beschicken und daß auch bei der Blutspende der Plasmasammelbeutel tiefer angebracht sein muß als der Blutsammelbeutel.

Um eine einfachere Handhabung zu ermöglichen, wurde das Schlauchsystem für die Beispiele 3 und 4 modifiziert.

Diese Versuche dienen gleichzeitig der Feststellung, ob bei der Zugabe der Vorfullungs-Lösung gemäß der Erfindung an einer separaten Anschlußstelle sich das System leichter handhaben läßt.

Wie aus diesen Beispielen ersichtlich ist, arbeitete unter Verwendung einer separat eingefüllten Vorfüllungs-Lösung und damit bei einfacherer Handhabung des Vorfüllvorganges sowie bei Anbringung des Plasmasammelbeutels tiefer als der Blutsammelbeutel dieses System einwandfrei.

Beispiel 5

Ein System bestehend aus:

Hollow fiber Modul zur Plasmatrennung (Plasmapur der Fa. Organon Technika)

Plasmabeutel zum Sammeln des Plasma - (Leerbeutel 500 ml Fa. Biotest)

wurde gemäß Fig. 6 installiert. Das System wurde mit isotoner NaCl-Lösung vorgefüllt. Ein mit stabilisiertem Blut gefüllter Beutel wurde über den Einstichdorn oberhalb der Plasmafiltereinheit angeschlossen und eine "Retransfusion" in einen Leerbeutel mit gleichzeitiger Plasmaabtrennung durchgeführt. Bei einem Höhenunterschied von 150 cm zwischen dem Blutbeutel und dem Leerbeutel wurden folgende Zeiten und Volumina gemessen:

|  | Versuch A | B |
|---|---|---|
| "Retransfusionszeit", Min. | 12 | 10 |
| Abgetrennte Plasmamenge, ml | 145 | 132 |

Wird die Spendezeit für Blut berücksichtigt, so ergibt sich eine Effektivität wie folgt:

| | |
|---|---|
| Spendezeit für 500 ml Blut | 5 Min. |
| Zeit für Retransfusion und | |
| Abtrennung des Plasmas | 11 Min. |
| Handhabung des Gerätes | 1 Min. |
| gesammeltes Plasma | 140 ml |

Dieser Zyklus kann z.B. 3 ˣ wiederholt werden, so daß man in ca. 50 Minuten einschließlich Retransfusion 420 ml Plasma erhält.

**Ansprüche**

1. Verfahren zur Gewinnung von Blutplasma bei einer Blutentnahme vom lebenden Körper, bei dem man nach einer Stauung der entnahmefähigen Blutgefäße mit Hilfe einer Blutentnahmekanüle Blut entnimmt und unter Ausnutzung von äußerem Druck und Schwerkraft und gegebenenfalls unter Verwendung einer Vorfüllungs-Lösung sowie nach Zusatz einer Stabilisatorlösung mit Hilfe eines Schlauches durch eine tiefer als die Blutquelle liegende Plasmafiltereinheit leitet, in der man Plasma vom Blut trennt, das abgetrennte Plasma einer Sammelstelle für Plasma und das partiell deplasmatisierte Blut einer davon getrennten Sammelstelle für Blut zuführt und gegebenenfalls das partiell deplasmatisierte Blut zum lebenden Körper in geänderter Schwerkraftlage durch dieselbe Plasmafiltereinheit retransfundiert, **dadurch gekennzeichnet,** daß man im Falle der Verwendung einer Vorfüllungs-Lösung diese mit Hilfe eines separaten Anschlusses zugibt, das gespendete Blutvolumen mißt, die Stabilitsatorlösung mit Hilfe einer Pumpe entsprechend dem gemessenen gespendeten Blutfluss bzw. Blutvolumen zudosiert, das Blut durch eine zwischen Blutentnahmestelle und Plasmafiltereinheit, jedoch nach dem Anschluß für die Stabilisatorlösung liegende Tropfkammer mit eingebautem Transfusionsfilter führt und die Sammelstelle für Plasma während des ganzen Verfahrens tiefer als die Sammelstelle für Blut anordnet.

2. Verfahren nach Anspruch 1, dadurch gekennzeichnet, daß die Messung des gespendeten Blutvolumens laufend erfolgt.

3. Verfahren nach einem der Ansprüche 1 oder 2, dadurch gekennzeichnet, daß die Messung des gespendeten Blutvolumens an der Schlauchaußenseite mit Hilfe eines Ultraschalldetektors erfolgt.

4. Verfahren zur Gewinnung von Blutplasma bei einer Blutentnahme vom lebenden Körper, bei dem man das System mit einer Vorfüllungs-Lösung vorfüllt, nach einer Stauung der entnahmefähigen Blutgefäße mit Hilfe einer Blutentnahmekanüle Blut entnimmt, mit Stabilisatorlösung vermischt und unter Ausnutzung von äußerem Druck und Schwerkraft mit Hilfe mindestens eines Schlauches durch eine tiefer als die Blutquelle liegende Plasmafiltereinheit leitet, in der man Plasma vom Blut trennt, das abgetrennte Plasma einer Sammelstelle für Plasma zuführt und das partiell deplasmatisierte Blut zum lebenden Körper retransfundiert, <u>dadurch gekennzeichnet</u>, daß eine bestimmte Menge Blut in mindestens einen Beutel, der eine auf die Blutmenge abgestimmte Menge Stabilisatorlösung enthält, gespendet wird, der Beutel mit dem stabilisierten Blut vom Beutelsystem abgetrennt und an das obere Ende eines Transfusiionsgerätes angeschlossen wird, das mit seinem unteren Ende mit dem Beutelsystem und der Spenderkanüle verbunden ist und das die Plasmafiltereinheit und die Sammelstelle für Plasma sowie eine Tropfkammer mit Transfusionsfilter enthält, das Blut über die Plasmafiltereinheit und die Tropfkammer dem Spender retransfundiert wird und während der Retransfusion das Plasma gesammelt wird.

5. Verfahren nach Anspruch 4, dadurch gekennzeichnet, daß die Vorfüllungs-Lösung mit Hilfe eines Einstichdorns am oberen Ende des Transfusionsgerätes dem System zugeführt wird.

6. Verfahren nach einem der vorstehenden Ansprüche, dadurch gekennzeichnet, daß man die Spende und die Retransfusion mehrere Male durchführt.

7. Vorrichtung zur Durchführung des Verfahrens nach einem der Ansprüche 1 bis 3, enthaltend eine Plasmafiltereinheit (4) zwischen einer Blutentnahmekanüle (1) und einer Sammelstelle für Blutplasma (5) sowie einer davon getrennten Sammelstelle für das partiell deplasmatisierte Blut (3) und einen Anschluß (11) für Stabilisatorlösung, <u>dadurch gekennzeichnet</u>, daß außerdem zwischen Injektionskanüle (1) und Plasmafiltereinheit (4) eine Tropfkammer mit eingebautem Transfusionsfilter (6), eine Dosierpumpe (8) zur Dosierung von Stabilisatorlösung (7), die mit einer Meßvorrichtung (9) für das gespendete Blutvolumen gekoppelt ist, und ein Anschluß (2) an Vorfüllungs-Lösung (10) angeordnet sind, wobei Anschluß (11) mit Dosierpumpe (8) und Stabilisatorlösung (7) sich vor der Tropfkammer mit eingebautem Transfusionsfilter - (6), bezogen auf die Richtung des Blutflusses bei der Spende, befinden.

8. Vorrichtung zur Durchführung des Verfahrens nach einem der Ansprüche 4 bis 6, enthaltend eine Blutentnahmekanüle (1), eine Plasmafiltereinheit (4) und eine Sammelstelle für Plasma (5), dadurch gekennzeichnet, daß sich die Blutentnahmekanule am Ende eines Beutelsystems (12) befindet, das mindestens einen mit Stabilisatorlösung zu füllenden Blutbeutel (13) enthält, und die Plasmafiltereinheit (4) sowie die Sammelstelle für Plasma - (5) sich innerhalb eines Transfusionsgerätes (20) befinden, das am oberen Ende einen oder mehrere Einstichdorne (14), im Anschluß an die Plasmafiltereinheit (4) in Richtung des Retransfundierungsflusses eine Tropfkammer mit eingebautem Transfusionsfilter (6) und am unteren Ende ein Verbindungssystem (18) zum Anschluß an das Beutelsystem (12) aufweist.

Fig. 1

Fig. 2

Fig. 3

Fig. 4

## Fig. 5

## Fig.6

13

14

15

4

150 cm

5

21

**EUROPÄISCHER TEILRECHERCHENBERICHT,**
der nach Regel 45 des Europäischen Patentübereinkommens für das weitere Verfahren als
europäischer Recherchenbericht gilt

Europäisches
Patentamt

Nummer der Anmeldung

## EINSCHLÄGIGE DOKUMENTE

EP 86104316.4

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | US - A - 4 498 983 (A.C.JILSTAD et al.)<br><br>* Gesamt; insbesondere Fig. 1,2, 9a,b; Spalte 4, Zeile 54 - Spalte 5, Zeile 63; Spalte 6, Zeilen 18-49; Spalte 13, Zeile 21 - Spalte 14, Zeile 56 *<br><br>-- | 7,8 | A 61 M 1/34<br>A 61 M 1/36 |
| A | EP - A1 - 0 085 016 (RHONE POULENC S.A.)<br><br>* Gesamt; insbesondere Fig. 2; Seite 7, Zeile 31 - Seite 12, Zeile 3 *<br><br>-- | 7,8 | |
| D,A | DE - A1 - 3 302 383 (M. LYSAGHT et al.)<br><br>* Gesamt *<br><br>-- | 7,8 | |

RECHERCHIERTE SACHGEBIETE (Int. Cl.4)

A 61 M 1/00

A 61 M 5/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich
ist, auf der Grundlage einiger Patentansprüche sinnvolle Ermittlungen über den Stand der Technik
durchzuführen.

Vollständig recherchierte Patentansprüche: 7,8

Unvollständig recherchierte Patentansprüche: —

Nicht recherchierte Patentansprüche: 1-6

Grund für die Beschränkung der Recherche:


(in Anwendung des Art. 52 (4))

| Recherchenort | Abschlußdatum der Recherche | Prüfer |
|---|---|---|
| WIEN | 17-09-1986 | LUDWIG |

KATEGORIE DER GENANNTEN DOKUMENTEN
X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder
nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1   03.82

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int. Cl.4) |
|---|---|---|---|
| A | GB - A - 2 112 293 (COBE LAB. INC.) | 7,8 | |
| | * Gesamt; insbesondere Fig. 1; Seite 2, Zeile 94 - Seite 3, Zeile 8 * | | |
| | -- | | |
| A | EP - A1 - 0 096 973 (KURARAY CO.) | 7,8 | |
| | * Gesamt; insbesondere Fig. 4; Seite 11, Zeile 18 - Seite 12, Zeile 15 * | | |
| | -- | | |
| A | US - A - 4 407 660 (CH. NEVENS et al.) | | RECHERCHIERTE SACHGEBIETE (Int. Cl.4) |
| | * Gesamt * | | |
| | -- | | |
| A | US - A - 3 945 380 (W.C.DABNEY et al.) | | |
| | * Gesamt * | | |
| | -- | | |
| A | US - A - 4 197 847 (I. DJERASSI) | | |
| | * Gesamt * | | |
| | -- | | |
| A | US - A - 4 309 993 (R.I. BROWN) | | |
| | * Spalte 1, Zeilen 6-38 * | | |
| | ---- | | |